# EUROPEAN PATENT APPLICATION

(11) **EP 4 809 924 A1**
(43) Date of publication of application: **23.09.2026**
(21) Application number: 25164158.5
(22) Date of filing: 17.03.2025
(51) Int. Cl.: A61B 5/339, A61B 5/00, A61B 5/318

(54) **SPATIAL AUDIO PRODUCTION FROM SONIFICATION OF ECG SIGNALS**

(71) Applicant: Koninklijke Philips N.V., 5656 AE Eindhoven (NL)
(72) Inventor: AN, Junmo, Eindhoven (NL); GREGG, Richard Earl, Eindhoven (NL)
(74) Representative: Philips Intellectual Property & Standards

(57) **Abstract**

A system for producing spatial audio from sonification of electrocardiogram (ECG) signals includes a sound player and a controller. The controller has a memory that stores instructions and a processor that executes the instructions. When executed by the processor, the instructions cause the system to: obtain ECG data; map the ECG data to sound as mapped ECG data; sonify the mapped ECG data; generate spatial audio based on sonifying the mapped ECG data by converting the mapped ECG data to spatial audio; and play the sound as spatial audio from the sound player.

## Description

### BACKGROUND OF THE INVENTION

Traditional methods of monitoring diagnostic electrocardiogram (ECG) signals rely on visual inspection of waveform data on monitors. While effective for expert users, visual monitoring of ECG waveform data for feedback is not particularly intuitive to non-expect users. Additionally, visual monitoring requires constant visual attention, which can be challenging in high-pressure environments such as surgeries or emergency care where clinicians must divide their focus among multiple tasks. The visual dependency can lead to fatigue and potential oversight of subtle yet critical changes in ECG patterns, resulting potentially in delayed response to cardiac abnormalities during patient care or treatment.

### SUMMARY OF THE INVENTION

According to an aspect of the present disclosure, a system for producing spatial audio from sonification of electrocardiogram (ECG) signals includes a sound player and a controller. The controller has a memory that stores instructions and a processor that executes the instructions. When executed by the processor, the instructions cause the system to: obtain ECG data; map the ECG data to sound as mapped ECG data; sonify the mapped ECG data; generate spatial audio based on sonifying the mapped ECG data by converting the mapped ECG data to spatial audio; and play the sound as spatial audio from the sound player.

According to another aspect of the present disclosure, a method for producing spatial audio from sonification of electrocardiogram (ECG) signals includes obtaining ECG data at a controller comprising a memory that stores instructions and a processor that executes the instructions; mapping, at the controller, the ECG data to sound as mapped ECG data; sonifying, at the controller, the mapped ECG data; generating, at the controller, spatial audio based on sonifying the mapped ECG data by converting the mapped ECG data to spatial audio; and playing the sound as spatial audio from a sound player.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 illustrates a system for spatial audio production from sonification of ECG signals, in accordance with a representative embodiment.
Fig. 2 illustrates a method for spatial audio production from sonification of ECG signals, in accordance with a representative embodiment.
Fig.. 3 illustrates another method for spatial audio production from sonification of ECG signals, in accordance with a representative embodiment.
Fig. 4A and Fig. 4B illustrate comparative waveforms of 12-lead ECG signals, in accordance with a representative embodiment.
Fig. 5A and Fig. 5B illustrate comparative generated audio waveforms from 12-lead ECG signals, in accordance with a representative embodiment.
Fig. 6A and Fig. 6B illustrate comparative single-sided amplitude spectrum of audio from 12-lead ECG signals, in accordance with a representative embodiment.
Fig. 7A and Fig. 7B illustrate comparative spectrograms of audio from 12-lead ECG signals, in accordance with a representative embodiment.
Fig. 8A and Fig. 8B illustrate comparative Mel spectrograms of audio from 12-lead ECG signals, in accordance with a representative embodiment.
Fig. 9A and Fig. 9B illustrate comparative continuous wavelet transforms of audio from 12-lead ECG signals, in accordance with a representative embodiment.
Fig. 10 illustrates a computer system, on which a method for spatial audio production from sonification of ECG signals is implemented, in accordance with another representative embodiment.

### DETAILED DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a,' 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, multi-lead ECG signals can be sonified and used to generate spatial audio. The spatial audio may be used to enhance cardiac monitoring and diagnosis. Auditory sonified multi-lead ECG signals may be converted into sound. By leveraging the human ear's ability to detect changes in sound, the techniques described herein aid in the early detection of abnormalities, continuous monitoring, and improved diagnosis. These techniques may be particularly useful in emergency situations, allowing for immediate auditory alerts and quicker intervention. The use of spatial audio is helpful for identifying specific areas of the heart affected by abnormalities, enhancing diagnostic accuracy. These techniques are also applicable to various cardiac conditions, providing a comprehensive tool for healthcare professionals to monitor and diagnose cardiac issues efficiently. The teachings herein facilitate the continuous and hands-free monitoring of ECG data, enabling healthcare professionals to detect various cardiac abnormalities quickly and accurately, even in high-pressure emergency situations.

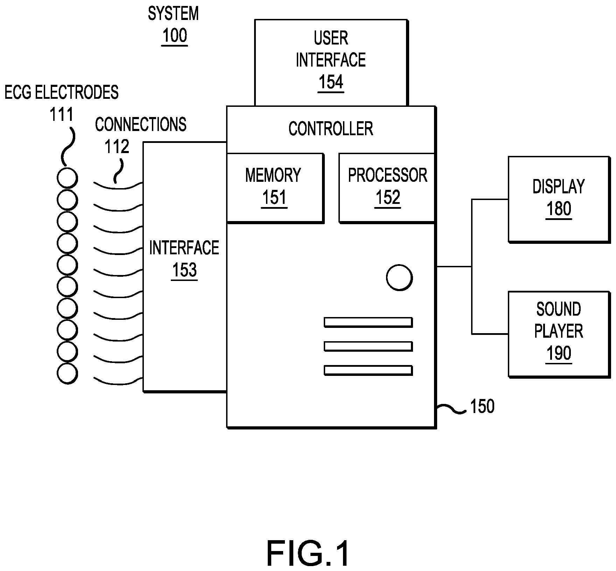

Fig. 1 illustrates a system 100 for spatial audio production from sonification of ECG signals, in accordance with a representative embodiment.

The system 100 in Fig. 1 is a system for spatial audio production from sonification of ECG signals and includes components that may be provided together in an integrated system or that may be distributed in multiple connected systems. The system 100 includes a controller 150, a display 180, a sound player 190, and ECG electrodes 111 connected by connections 112 to an interface 153 of the controller 150. The controller 150 includes a memory 151 that stores instructions, a processor 152 that executes the instructions, the interface 153 for the ECG electrodes 111, and a user interface 154.

ECG electrodes 111 are sensors placed on the body to detect electrical signals. Leads are the electrical signals or perspectives captured from the ECG electrodes 111. The leads are transmitted to the interface 153 via connections 112. An example of the connections 112 is cables.

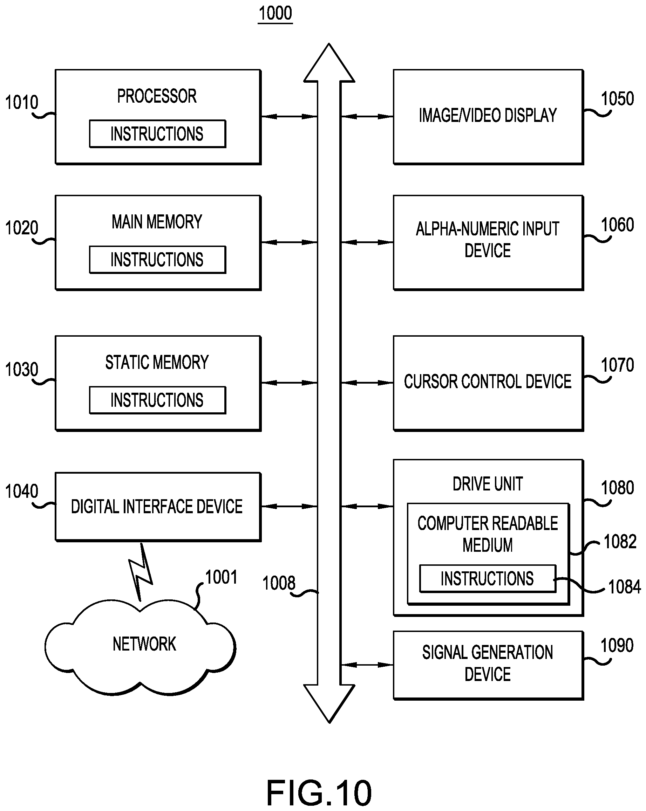

The controller 150 includes at least the memory 151 that stores instructions and the processor 152 that executes the instructions. A computer that can be used to implement the controller 150 is depicted in Fig. 10, though a controller 150 may include more or fewer elements than depicted in Fig. 1 or Fig. 10. In some embodiments, multiple different elements of the system 100 in Fig. 1 may include a controller such as the controller 150. For example, either or both of the display 180 or the sound player 190 may include an embedded controller. The user interface 154 may include a display separate from the display 180, as well as input mechanisms such as buttons, keys, knobs, a mouse, a microphone or other interactive elements by which a user can provide inquiries or instructions to the system 100.

The display 180 may be connected to the controller 150 via a local wired interface such as an Ethernet cable or via a local wireless interface such as a Wi-Fi connection. The display 180 may be interfaced with other user input devices by which users can input instructions, including mouses, keyboards, thumbwheels and so on. The display 180 may be a monitor such as a computer monitor, a display on a mobile device, an augmented reality display, a television, an electronic whiteboard, or another screen configured to display electronic imagery. The display 180 may also include one or more input interface(s) that may connect to other elements or components, as well as an interactive touch screen configured to display prompts to users and collect touch input from users. According to the teachings herein, even when the display 180 is provided in the same physical setting as the ECG electrodes 111 and the controller 150 and used to visually output waveforms based on the ECG data, the sound played as spatial audio as the result of the controller 150 executing instructions to generate the spatial audio is played by the sound player 190. In some embodiments, however, the sound player 190 may be integrated with or in the display 180. Therefore, the sound played by the sound player 190 based on the ECG data may be played with or without a visual display of the ECG waveforms based on the ECG data.

The controller 150 may include additional interfaces, such as a first additional interface, a second additional interface, a third additional interface, and a fourth additional interface. One or more of the interface 153, the user interface 154 or any additional interface may include ports, disk drives, wireless antennas, or other types of receiver circuitry that connect the controller 150 to other electronic elements. One or more of the interface 153 or any additional interface may also include user interfaces such as buttons, keys, a mouse, a microphone, a speaker, a display separate from the display 180, or other elements that users can use to interact with the controller 150 such as to enter instructions and receive output.

The controller 150 may perform some of the operations described herein directly and may implement other operations described herein indirectly. For example, the controller 150 may indirectly control operations such as by generating and transmitting content to be displayed on the display 180 or output as sound by the sound player 190. The controller 150 may directly control other operations such as logical operations performed by the processor 152 executing instructions from the memory 151 based on input received from electronic elements such as the ECG electrodes 111 as well as input received users via the user interface 154 or another user interface. Accordingly, the processes implemented by the controller 150 when the processor 152 executes instructions from the memory 151 may include steps not directly performed by the controller 150.

The sound player 190 is representative of one or more speakers. The sound player 190 may be, for example, left-right speakers of a headset, earphones, or earbuds. The sound player 190 may also be a built-in speaker, including a built-in speaker that is an element of the display 180 or another monitor. Additionally, the sound player 190 may also be a stand-alone dedicated speaker that is not an element of a monitor, or other audio output devices such as portable Bluetooth speakers or soundbars.

The controller 150 obtains ECG data from the ECG electrodes via the connections 112 and the interface 153. The controller 150 executes instructions to map the ECG data to sound as mapped ECG data; sonify the mapped ECG data; generate spatial audio based on sonifying the mapped ECG data by converting the mapped ECG data to spatial audio; and play the sound via the sound player 190 as spatial audio from the sound player. Sonification involves converting data into sound. Sonification using the system 100 leverages the human ear's sensitivity to changes in auditory patterns. The human auditory system is adept at detecting even minor variations in sound, and thus may be a valuable tool for monitoring ECG signals. Sonification resulting in sound from the sound player 190 enables eyes-free monitoring, allowing healthcare professionals to perceive and respond to cardiac abnormalities without the need to constantly look at a monitor. This auditory approach, when combined with spatial audio techniques, may enhance the ability to detect anomalies more efficiently, identify the exact location of disease or abnormality in the heart, facilitate continuous monitoring, and provide real-time alerts in high-stress, multitasking environments. Sonification may be particularly beneficial in scenarios where visual attention is divided or when visual displays such as the display 180 are not feasible. For instance, in emergency situations or during surgical procedures, healthcare professionals can rely on auditory cues to monitor patients' cardiac activity, ensuring timely interventions. Additionally, the use of sonification can aid in training and education, helping medical students and professionals develop a keen sense of auditory detection for cardiac irregularities. By integrating sonification into healthcare practices, patient outcomes can be improved, and cognitive load on healthcare providers can be reduced, enhancing overall efficiency of cardiac monitoring systems. By converting ECG data into spatial audio, healthcare professionals improve intuitive understanding of cardiac activity.

Sonification with spatial audio can aid in the early detection of various abnormalities, such as ST-elevation, which is crucial for diagnosing conditions like myocardial infarction with precise localization in the heart. The human ear is proficient at detecting changes and patterns in sound, even in noisy environments. As another example, sonification with spatial audio allows healthcare professionals to monitor ECG data while focusing on other tasks, as auditory signals can be processed in the background, enabling multitasking. As yet another example, using sonification methods with spatial audio may assist in accurately classifying the severity and precise location of cardiac conditions during monitoring. Polarity sonification may be used to achieve high accuracy in detecting transitions from healthy to unhealthy states.

Sonification can also be designed to be informative so that the teachings herein may be used by both experts and non-experts. Water ambiance sonification has been found to be aesthetically pleasing and effective for long-term listening. Understanding the spatial nature of ECG visually from multiple waveforms takes long experience but spatial sound is intuitive. Additionally, users may use the system 100 to interact with sonified data, adjusting parameters to highlight specific patterns or anomalies. This interactivity can enhance the saliency of important ECG features. Any abnormal sounds may alert healthcare professionals instantly to potential issues, allowing for quicker intervention. The system 100 also allows hands-free and eyes-free monitoring. By eliminating the need for constant visual attention even in the presence of the display 180, clinicians can efficiently monitor ECG data while multitasking or performing tasks that require visual focus, such as surgeries or emergency care, and this can reduce visual fatigue and improve overall efficiency.

Additionally, while sonification of the signal with spatial audio using the system 100 is described herein primarily in the context of ECG, these teachings may also be used for physiological waveform signals, including oxygen saturation (SpO2), pulse oximetry/ photoplethysmography (PPG), carbon dioxide levels (CO2), capnography, invasive blood pressure (IBP), non-invasive blood pressure (NIBP), respiratory signal, plethysmogram (pleth wave), and others (like electroencephalogram (EEG), electromyogram (EMG), echocardiogram, doppler ultrasound, etc.).

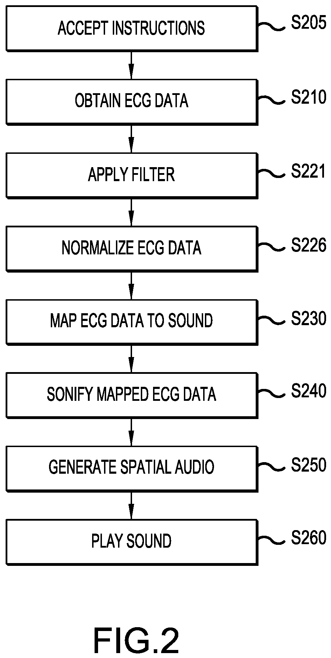

Fig. 2 illustrates a method for spatial audio production from sonification of ECG signals, in accordance with a representative embodiment.

The method of Fig. 2 may be performed by the system 100 including the controller 150.

At S205, instructions are accepted such as via the user interface 154. The instructions may include instructions to adjust parameters for sonifying mapped ECG data (not yet obtained).

At S210, ECG data is obtained. The ECG data is obtained by the controller 150 via the ECG electrodes 111. While most embodiments herein involve real-time ECG data obtained live from the ECG electrodes 111, in some embodiments the ECG data may be obtained at S210 from a memory such as when the ECG data is being used for training. However, in most embodiments the ECG data is obtained at S210 live such as in a medical facility or an ambulance.

An ECG data format may comprise a digital structure used to store and transmit electrocardiogram (ECG) data, typically including electrical signals recorded from the heart over time. The electrical signals may be used to generate the familiar waveforms visualized on ECG displays. An ECG data format may also include patient information and annotations such as wave measurements and intervals. An ECG data format may be an example or type of a standardized format such as DICOM (Digital Imaging and Communications in Medicine) or HL7 aECG (Health Level Seven Annotated Electrocardiogram) to ensure interoperability between different devices and systems.

At S221, a filter is applied to the ECG data. The filter may be applied in preprocessing to remove noise and artifacts from the ECG data obtained at S210.

At S226, the ECG data is normalized. Normalization may also be performed during preprocessing to scale the ECG signal to a standard range, ensuring consistency for sound mapping at S230 using appropriate normalization methods.

At S230, the ECG data is mapped to sound. Mapping may include mapping of characteristics of or from the ECG signal (e.g., heart rate, amplitude, frequency components) to characteristics of sound (e.g., tempo from the heart rate, volume or intensity from the amplitude, pitches or tones from the frequency components).

At S240, the mapped ECG data is sonified. Sonification involves translation of electrical heart signals into audible sounds. Sonification at S240 may be based on a selection among available sonification methods according to the instructions accepted at S205.

At S250, spatial audio is generated based on the sonified mapped ECG data. The generation of spatial audio at S250 may involve synthesis of sound with spatial audio, and may include generating a base sound, producing spatial audio that correlates with anatomical position of a cardiac anomaly, and one of several possible spatial mapping configurations described later.

At S260, sound of the spatial audio is played via the sound player 190. The sound of the spatial audio played at S260 is acquired via multi-lead ECG in the system 100 in Fig. 1. Spatial audio playback at S260 may involve playing generated sounds through speakers or headphones. The user may listen for patterns or anomalies in the sound that correspond to irregularities in the ECG signal. In some embodiments, spatial audio may be played in six positions using a left-right headset as the sound player 190. The user may be enabled to select the preferred sonification methods using the user interface 154 or another user interface. Options for sonification methods may be provided, including water ambience, polarity, morph, polyphonic, stethoscope, and other. A full sound system or a headset with multiple drivers can also be used. The six positions are as follows:
i. Front-center position: This is the sound coming from both earbuds equally, and is therefore the easiest to achieve.
ii. Bottom-center position: To simulate this, the sound may be played slightly louder in the left ear than the right, creating a perceived downward tilt.
iii. Left-side position: The sound may be played almost exclusively in the left ear, creating a strong leftward bias.
iv. Center position: This is similar to the front-center position, but with a slight delay in the sound reaching the left ear compared to the right, creating a perceived center positioning.
v. Rear-center position: The sound may be played slightly louder in the right ear than the left, with a delay in the sound reaching the right ear compared to the left, creating a perceived rearward tilt.
vi. Right-side position: The sound may be played almost exclusively in the right ear, creating a strong rightward bias.

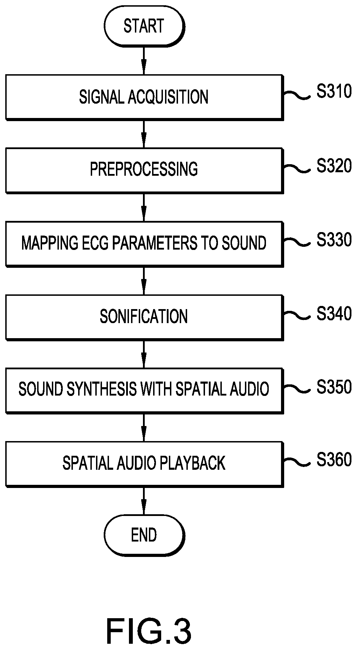

Sonifying multi-lead ECG signals with spatial sound as in Fig. 2 and Fig. 3 offers significant benefits for cardiac monitoring and diagnosis. By utilizing spatial audio, this approach correlates auditory cues with the anatomical position of cardiac anomalies, employing spatialization techniques to place sounds within a three-dimensional space. This spatial representation aids in the early detection of various abnormalities, such as ST-elevation, crucial for diagnosing myocardial infarction, as the human ear is adept at recognizing changes and patterns in sound, even in noisy environments.

Sonification allows healthcare professionals to continuously monitor ECG data while attending to other tasks, processing auditory signals in the background and facilitating multitasking. Various sonification methods enhance the accuracy of diagnosing cardiac conditions. For example, polarity sonification has proven effective in identifying transitions from healthy to unhealthy states. Designed to be user-friendly, sonification offers various methods such as water ambience, polarity, morph, stethoscope, and others. These methods can inform both experts and non-experts. For example, water ambience sonification is aesthetically pleasing and suitable for long-term listening.

The interactive nature of this technology enables users to adjust parameters, highlighting specific patterns or anomalies, thereby improving the saliency of important ECG features. By placing sounds in a three-dimensional auditory space, clinicians can more easily locate and identify the source of anomalies, enhancing their ability to respond promptly to potential issues. This feature is particularly beneficial in situations requiring visual attention elsewhere, such as during surgeries or emergency care. Overall, spatial audio not only allows clinicians to focus on other critical tasks without constantly monitoring visual displays, but it also reduces visual fatigue and minimizes the risk of missing important changes, thereby enhancing efficiency and effectiveness in cardiac care.

Fig. 3 illustrates another method for spatial audio production from sonification of ECG signals, in accordance with a representative embodiment.

Fig. 3 shows a flowchart of the procedure of ECG sonification with spatial audio. At S310, signal acquisition is performed. Signal acquisition at S310 may involve collecting ECG data by obtaining an ECG signal using the ECG electrodes 111. S310 may also involve ensuring the ECG data is clean, such as by ensuring the ECG data complies with an expected format.

At S320, preprocessing is performed. Preprocessing at S320 may involve filtering by applying one or more filters to remove noise and artifacts from the ECG signal, such as noise above a first (upper) frequency threshold or below a second (lower) frequency threshold. S320 may also involve normalization by normalizing the ECG signal to a standard range to ensure consistent sound.

At S330, the method of Fig. 3 includes mapping ECG parameters to sound. Heart rate may be mapped to the tempo of the sound. The amplitude of the ECG signal may be used to control the volume or intensity of the sound. Frequency components of the ECG signal may be mapped to different pitches or tones. Mapping of the ECG data to sound at S330 may include mapping heart rate to tempo of the sound, controlling volume of the sound based on amplitude of the ECG data, and mapping frequency components of the ECG data to tones.

At S340, sonification is performed. ECG sonification is a technique used for 12-lead ECGs. ECG sonification involves translating electrical heart signals into audible sounds. The auditory representation of the electrical heart signals facilitates monitoring and diagnosing cardiac conditions. Sonification methods include water ambience, polarity, morph, polyphonic, stethoscope, and more. Water ambience involves transforming heart activity into ambient water sounds insofar as deviations from normal heartbeats create distinct water sound patterns. Polarity uses a formant synthesizer to map ECG parameters to sound parameters, representing changes in the ECG signal with changes in sound polarity. Morph uses timbre morphing to highlight changes in frequency, rhythm, or amplitude, allowing quick assessment of abnormalities. Polyphonic sonification involves simultaneously sonifying multiple ECG leads, each represented by a different instrument or sound, to create a polyphonic soundscape. Users can select their preferred scale, such as C major, C minor, D major, D minor, or various other scales, using the user interface 154. Stethoscope sonification involves combining the ECG signal with a stethoscope recording, modulating the stethoscope sound to indicate abnormalities.

At S350, sound synthesis with spatial audio is performed. Sound synthesis with spatial audio may involve several separate functions. An initial function may be to generate base sound using sound synthesis techniques to generate the corresponding sounds based on the mapped parameters such as frequency, amplitude, and waveform type. Another function may be production of spatial audio that correlates with anatomical positions of a cardiac anomaly using spatialization techniques to place the generated sounds in a three-dimensional (3D) space. Spatial mapping in standard 12-lead ECG involves assigning specific spatial audio positions to different regions of the heart based on the standard 12-lead ECG. A 12-lead ECG layout may be arranged according to Table I as follows:

| | | | |
|---|---|---|---|
| I Lateral | aVR | V1 Septal | V4 Anterior |
| II Inferior | aVL Lateral | V2 Septal | V5 Lateral |
| III Inferior | aVF Inferior | V3 Anterior | V6 Lateral |

For example, myocardial infarction (MI) may be detected using the following configuration:
i. Anterior MI (Leads V3 and V4): Front-center position.
ii. Inferior MI (Leads II, aVF, and III): Bottom-center position.
iii. Lateral MI (Leads I, aVL, V5, and V6): Left-side position.
iv. Septal MI (Leads V1 and V2): Center position.
v. Posterior MI (additional leads such as leads V7, V8, and V9): Rear-center position.
vi. Right Ventricular Infarction (additional right-sided leads like V3R to V6R): Right-side position.

The spatial audio generated at S250 or S350 in this configuration is generated by correlating anatomical position of a cardiac anomaly to place sounds in a three-dimensional space by mapping spatial audio positions of electrodes of a 12-lead ECG to different regions of a heart according to i, ii, iii and iv, and additional leads to additional regions relative to the heart according to v and vi.

Another function may be spatial mapping in standard 12-lead ECG in Cabrera lead order. The Cabrera lead order reorganizes the limb leads to present a more intuitive sequence of electrical activity. It arranges the leads anatomically as follows: aVL, I, -aVR, II, aVF, and III, positioning leads with opposing vectors next to each other. Spatial mapping in Cabrera lead order involves assigning specific spatial audio positions based on this arrangement. For example, spatial mapping in Cabrera lead order may involve the following configuration:
i. Anterior MI (Leads V3 and V4): Front-center position.
ii. Inferior MI (Leads II, aVF, and III): Bottom-center position.
iii. Lateral MI (Leads I, aVL, -aVR, V5, and V6): Left-side position.
iv. Septal MI (Leads V1 and V2): Center position.
v. Posterior MI (additional leads such as leads V7, V8, and V9): Rear-center position.
vi. Right Ventricular Infarction (additional right-sided leads like V3R to V6R): Right-side position.

The spatial audio generated at S250 or S350 in this configuration is generated by correlating anatomical position of a cardiac anomaly to place sounds in a three-dimensional space by mapping spatial audio positions of electrodes of a 12-lead ECG in a Cabrera lead order according to i, ii, iii and iv, and additional leads to additional regions relative to a heart according to v and vi.

Another function may be spatial mapping by converting the standard 12-lead ECG into Frank X, Y, Z spatial leads. The Frank lead system is a vectorcardiographic technique that places electrodes on the body to generate three orthogonal leads (X, Y, and Z). These leads represent the heart's electrical activity in a three-dimensional space. Specifically, the X lead corresponds to the left-right axis, the Y lead to the head-to-foot axis, and the Z lead to the front-back axis. For example, spatial mapping by conversion in this manner may involve the following configuration:
i. X signal corresponds to lateral left-right spatial orientation
ii. Y signal corresponds to superior up-down spatial orientation
iii. Z signal corresponds to anterior front-back spatial orientation
iv. Mapping the 12-lead signal to Frank leads assumes the signal is generated by a single spatial dipole. The model is a single dipole generator. The accuracy of the model can be improved by modeling with more than one spatial dipole generator. In that way, the sound is generated as a weighted summation of the sound from each spatial dipole generator.

At S360, spatial audio playback is performed. The sound player 190 may produce sounds corresponding to the synthesized sound from S350. That spatial audio playback reflects the three-dimensional positioning of the ECG electrodes 111. Observers may be trained to develop a keen sense of auditory detection for cardiac irregularities. At S360, sound of the spatial audio is played via the sound player 190. The sound of the spatial audio played at S360 is acquired via multi-lead ECG in the system 100 in Fig. 1. Spatial audio playback at S360 may involve playing generated sounds through speakers or headphones. The user may listen for patterns or anomalies in the sound that correspond to irregularities in the ECG signal. In some embodiments, spatial audio may be played in six positions using a left-right headset as the sound player 190. The user may be enabled to select the preferred sonification methods using the user interface 154 or another user interface. Options for sonification methods may be provided, including water ambience, polarity, morph, polyphonic, stethoscope, and other techniques. A full sound system or a headset with multiple drivers can also be used.

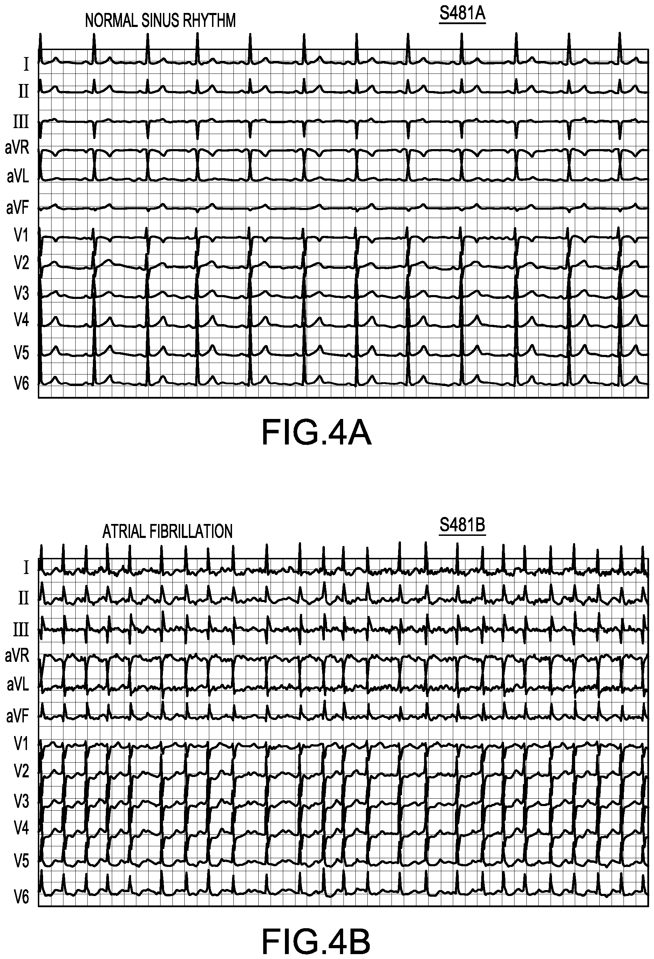

Fig. 4A and Fig. 4B illustrate comparative waveforms of 12-lead ECG signals, in accordance with a representative embodiment.
Fig. 4A and Fig. 4B show waveforms of 12-lead ECG signals including (Fig. 4A) normal sinus rhythm and (Fig. 4B) atrial fibrillation.

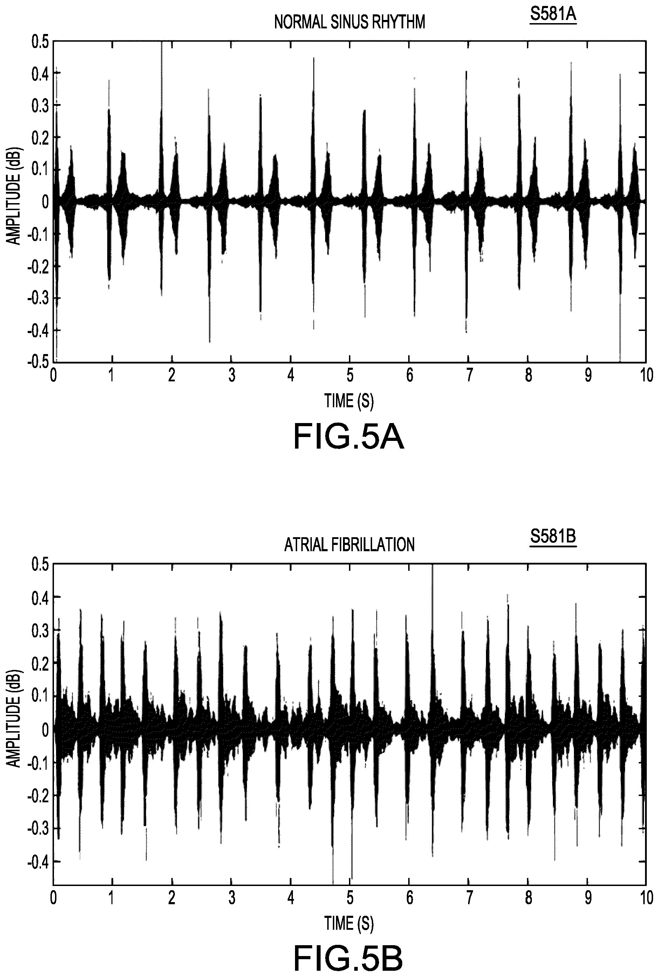

Fig. 5A and Fig. 5B illustrate comparative generated audio waveforms from 12-lead ECG signals, in accordance with a representative embodiment.
Fig. 5A and Fig. 5B show generated audio waveforms from 12-lead ECG signals including (Fig. 5A) normal sinus rhythm and (Fig. 5B) atrial fibrillation.

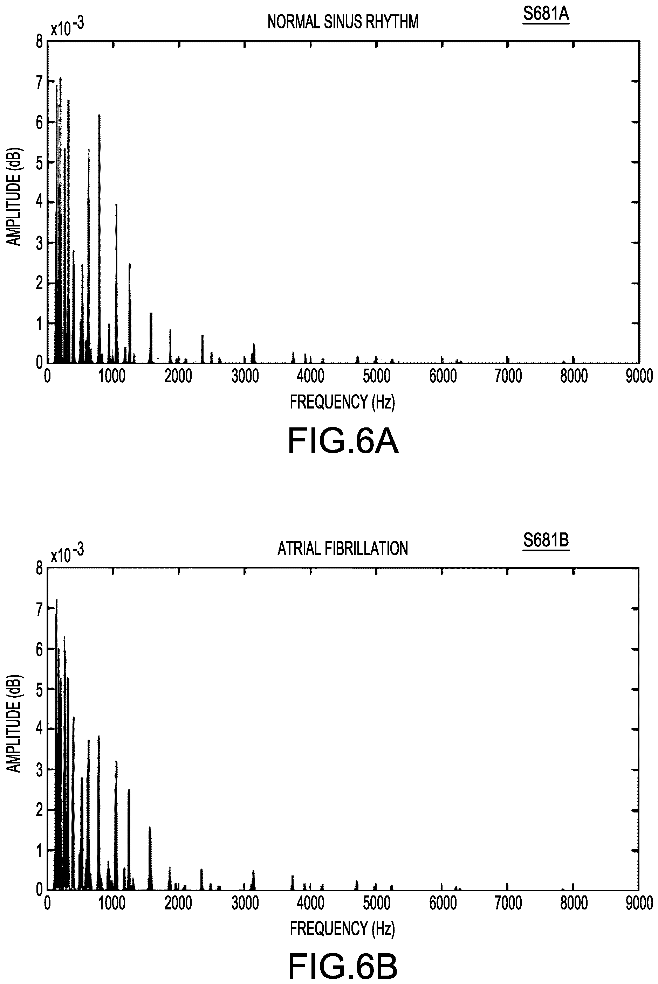

Fig. 6A and Fig. 6B illustrate comparative single-sided amplitude spectrum of audio from 12-lead ECG signals, in accordance with a representative embodiment.
Fig. 6A and Fig. 6B show single-sided amplitude spectrum of audio from 12-lead ECG signals including (Fig. 6A) normal sinus rhythm and (Fig. 6B) atrial fibrillation.

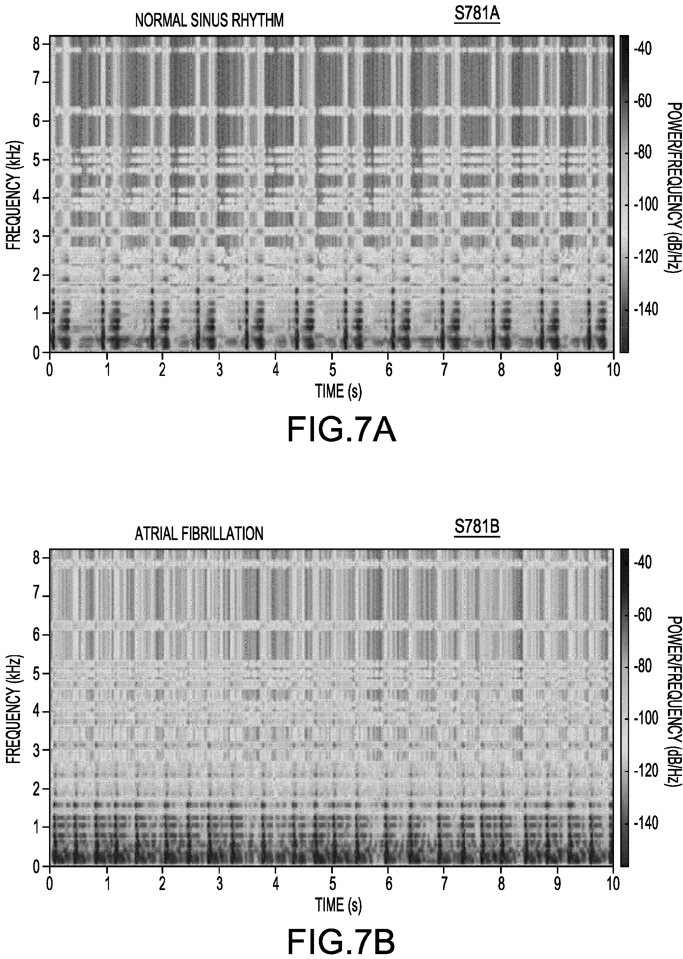

Fig. 7A and Fig. 7B illustrates comparative spectrograms of audio from 12-lead ECG signals, in accordance with a representative embodiment.
Fig. 7A and Fig. 7B show spectrograms of audio from 12-lead ECG signals including (Fig. 7A) normal sinus rhythm and (Fig. 7B) atrial fibrillation.

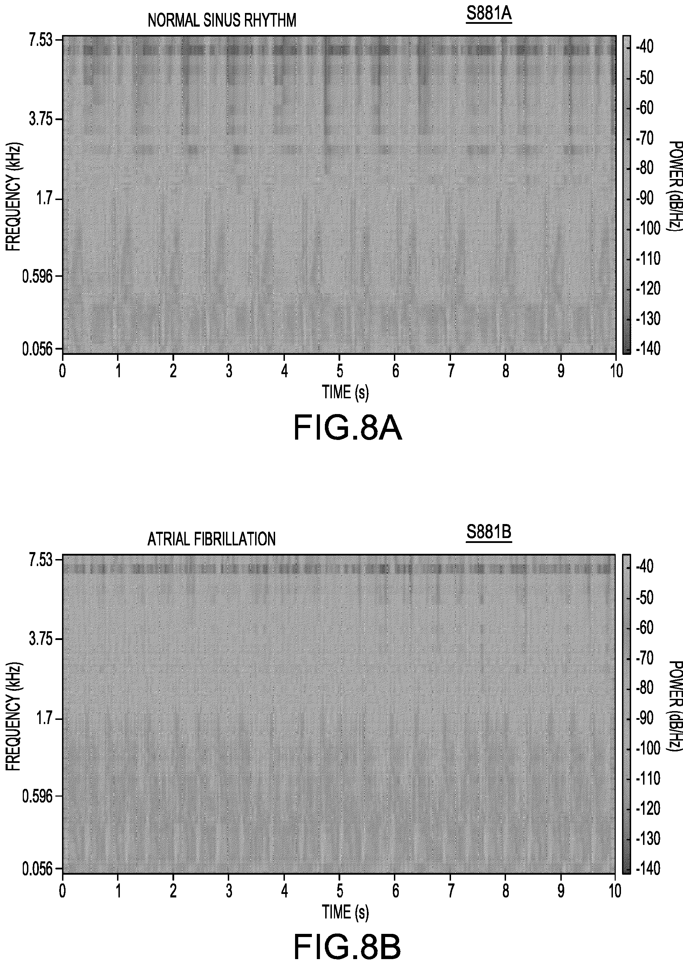

Fig. 8A and Fig. 8B illustrate comparative Mel spectrograms of audio from 12-lead ECG signals, in accordance with a representative embodiment.
Fig. 8A and Fig. 8B show Mel spectrogram of audio from 12-lead ECG signals including (Fig. 8A) normal sinus rhythm and (Fig. 8B) atrial fibrillation.

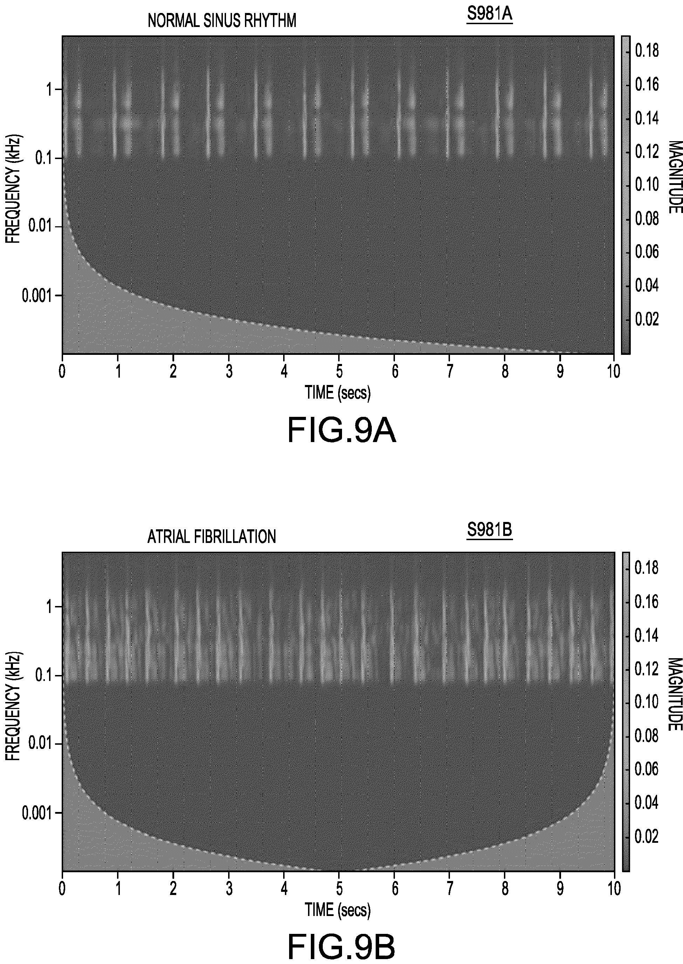

Fig. 9A and Fig. 9B illustrate comparative continuous wavelet transforms of audio from 12-lead ECG signals, in accordance with a representative embodiment.
Fig. 9A and Fig. 9B show continuous wavelet transform of audio from 12-lead ECG signals including (Fig. 9A) normal sinus rhythm and (Fig. 9B) atrial fibrillation.
Fig. 10 illustrates a computer system, on which a method for spatial audio production from sonification of ECG signals is implemented, in accordance with another representative embodiment.

Referring to Fig. 10, the computer system 1000 includes a set of software instructions that can be executed to cause the computer system 1000 to perform any of the methods or computer-based functions disclosed herein. The computer system 1000 may operate as a standalone device or may be connected, for example, using a network 1001, to other computer systems or peripheral devices. In embodiments, a computer system 1000 performs logical processing based on digital signals received via an analog-to-digital converter.

In a networked deployment, the computer system 1000 operates in the capacity of a server or as a client user computer in a server-client user network environment, or as a peer computer system in a peer-to-peer (or distributed) network environment. The computer system 1000 can also be implemented as or incorporated into various devices, such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The computer system 1000 can be incorporated as or in a device that in turn is in an integrated system that includes additional devices. In an embodiment, the computer system 1000 can be implemented using electronic devices that provide voice, video or data communication. Further, while the computer system 1000 is illustrated in the singular, the term "system" shall also be taken to include any collection of systems or sub-systems that individually or jointly execute a set, or multiple sets, of software instructions to perform one or more computer functions.

As illustrated in Fig. 10, the computer system 1000 includes a processor 1010. The processor 1010 may be considered a representative example of a processor of a controller and executes instructions to implement some or all aspects of methods and processes described herein. The processor 1010 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 1010 is an article of manufacture and/or a machine component. The processor 1010 is configured to execute software instructions to perform functions as described in the various embodiments herein. The processor 1010 may be a general-purpose processor or may be part of an application specific integrated circuit (ASIC). The processor 1010 may also be a microprocessor, a microcomputer, a processor chip, a controller, a microcontroller, a digital signal processor (DSP), a state machine, or a programmable logic device. The processor 1010 may also be a logical circuit, including a programmable gate array (PGA), such as a field programmable gate array (FPGA), or another type of circuit that includes discrete gate and/or transistor logic. The processor 1010 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 1000 further includes a main memory 1020 and a static memory 1030, where memories in the computer system 1000 communicate with each other and the processor 1010 via a bus 1008. Either or both of the main memory 1020 and the static memory 1030 may be considered representative examples of a memory of a controller, and store instructions used to implement some, or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 1020 and the static memory 1030 are articles of manufacture and/or machine components. The main memory 1020 and the static memory 1030 are computer-readable mediums from which data and executable software instructions can be read by a computer (e.g., the processor 1010). Each of the main memory 1020 and the static memory 1030 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 1000 further includes a video display unit 1050, such as a liquid crystal display (LCD), an organic light emitting diode (OLED), a flat panel display, a solid-state display, or a cathode ray tube (CRT), for example. Additionally, the computer system 1000 includes an input device 1060, such as a keyboard/virtual keyboard or touch-sensitive input screen or speech input with speech recognition, and a cursor control device 1070, such as a mouse or touch-sensitive input screen or pad. The computer system 1000 also optionally includes a disk drive unit 1080, a signal generation device 1090, such as a speaker or remote control, and/or a network interface device 1040.

In an embodiment, as depicted in Fig. 10, the disk drive unit 1080 includes a computer-readable medium 1082 in which one or more sets of software instructions 1084 (software) are embedded. The sets of software instructions 1084 are read from the computer-readable medium 1082 to be executed by the processor 1010. Further, the software instructions 1084, when executed by the processor 1010, perform one or more steps of the methods and processes as described herein. In an embodiment, the software instructions 1084 reside all or in part within the main memory 1020, the static memory 1030 and/or the processor 1010 during execution by the computer system 1000. Further, the computer-readable medium 1082 may include software instructions 1084 or receive and execute software instructions 1084 responsive to a propagated signal, so that a device connected to a network 1001 communicates voice, video or data over the network 1001. The software instructions 1084 may be transmitted or received over the network 1001 via the network interface device 1040.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, spatial audio production from sonification of ECG signals enables generation of spatial audio which may be used to enhance cardiac monitoring and diagnosis. Auditory sonified multi-lead ECG signals may be converted into sound. By leveraging the human ear's ability to detect changes in sound, the techniques described herein aid in the early detection of abnormalities, continuous monitoring, and improved diagnosis. These techniques may be particularly useful in emergency situations, allowing for immediate auditory alerts and quicker intervention. The use of spatial audio is helpful for identifying specific areas of the heart affected by abnormalities, enhancing diagnostic accuracy. These techniques are also applicable to various cardiac conditions, providing a comprehensive tool for healthcare professionals to monitor and diagnose cardiac issues efficiently. The teachings herein facilitate the continuous and hands-free monitoring of ECG data, enabling healthcare professionals to detect various cardiac abnormalities quickly and accurately, even in high-pressure emergency situations.

The teachings herein based on spatial audio production from sonification of ECG signals may be used in a variety of applications. Such applications include diagnostic ECG using cardiographs, stress testing systems, portable/bedside patient monitors, continuous patient monitoring systems including those used for prehospital care including in ambulances, patient wearable monitors, and ventilators.

Although spatial audio production from sonification of ECG signals has been described with reference to several exemplary embodiments, it is understood that the words that have been used are words of description and illustration, rather than words of limitation. Changes may be made within the purview of the appended claims, as presently stated and as amended, without departing from the scope and spirit of spatial audio production from sonification of ECG signals in its aspects. Although spatial audio production from sonification of ECG signals has been described with reference to particular means, materials and embodiments, spatial audio production from sonification of ECG signals is not intended to be limited to the particulars disclosed; rather spatial audio production from sonification of ECG signals extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A system for producing spatial audio from sonification of electrocardiogram (ECG) signals, comprising:
a sound player (190); and
a controller (150) comprising a memory (151) that stores instructions, and a processor (152) that executes the instructions, wherein, when executed by the processor, the instructions cause the system to:
obtain (S210) ECG data;
map (S230) the ECG data to sound as mapped ECG data;
sonify (S240) the mapped ECG data;
generate (S250) spatial audio based on sonifying the mapped ECG data by converting the mapped ECG data to spatial audio; and
play (S260) the sound as spatial audio from the sound player (190).

2. The system of claim 1, wherein the sound is played as spatial audio from the sound player (190) in real-time as the ECG data is acquired via multi-lead ECG during a procedure.

3. The system of claim 1, wherein, when executed by the processor, the instructions further cause the system to:
process the ECG data before mapping the ECG data to sound by:
applying (S221) a filter to remove noise and artifacts from the ECG data; and
normalizing (S226) the ECG data to a standard range to ensure consistent sound mapping.

4. The system of claim 1, further comprising:
an interface (154), wherein, when executed by the processor, the instructions further cause the system to:
accept (S205) instructions via the interface (154) to adjust parameters for sonifying the mapped ECG data, and sonify the mapped ECG data based on adjusting the parameters according to the instructions.

5. The system of claim 1, wherein mapping the ECG data to sound comprises:
mapping heart rate to tempo of the sound;
controlling volume of the sound based on amplitude of the ECG data; and
mapping frequency components of the ECG data to tones.

6. The system of claim 1, wherein playing the sound as spatial audio from the sound player (190) comprises playing audio in each of a plurality of positions in a left-right headset.

7. A method for producing spatial audio from sonification of electrocardiogram (ECG) signals, the method comprising:
obtaining (S210) ECG data at a controller (150) comprising a memory (151) that stores instructions and a processor (152) that executes the instructions;
mapping (S230), at the controller (150), the ECG data to sound as mapped ECG data;
sonifying (S240), at the controller (150), the mapped ECG data;
generating (S250), at the controller (150), spatial audio based on sonifying the mapped ECG data by converting the mapped ECG data to spatial audio; and
playing (S260) the sound as spatial audio from a sound player (190).

8. The method of claim 7, wherein the sound is played as spatial audio from the sound player (190) in real-time as the ECG data is acquired via multi-lead ECG during a procedure.

9. The method of claim 7, further comprising: processing the ECG data before mapping the ECG data to sound by: applying (S221) a filter to remove noise and artifacts from the ECG data; and normalizing (S226) the ECG data to a standard range to ensure consistent sound mapping.

10. The method of claim 7, further comprising:
accepting (S205), at an interface (154), instructions to adjust parameters for sonifying the mapped ECG data;
adjusting the parameters based on the instructions; and
sonifying the mapped ECG data based on adjusting the parameters according to the instructions.

11. The method of claim 7, wherein mapping the ECG data to sound comprises:
mapping heart rate to tempo of the sound;
controlling volume of the sound based on amplitude of the ECG data; and mapping frequency components of the ECG data to tones.

12. The method of claim 7, further comprising:
generating a base sound based on the mapped ECG data to sonify the mapped ECG data; and
generating the spatial audio by correlating anatomical position of a cardiac anomaly to place sounds in a three-dimensional space by mapping spatial audio positions of electrodes of a 12-lead ECG to different regions of a heart and additional leads to additional regions relative to the heart.

13. The method of claim 7, further comprising:
generating a base sound based on the mapped ECG data to sonify the mapped ECG data; and
generating the spatial audio by correlating anatomical position of a cardiac anomaly to place sounds in a three-dimensional space by mapping spatial audio positions of electrodes of a 12-lead ECG in a Cabrera lead order and additional leads to additional regions relative to a heart.

14. The method of claim 7, further comprising:
generating a base sound based on the mapped ECG data to sonify the mapped ECG data; and
generating the spatial audio by correlating anatomical position of a cardiac anomaly to place sounds in a three-dimensional space by mapping spatial audio positions of electrodes of a 12-lead ECG to Frank X, Y, Z spatial leads.

15. The method of claim 7, wherein playing the sound as spatial audio from the sound player (190) comprises playing audio in each of a plurality of positions in a left-right headset.
